# EUROPÄISCHE PATENTSCHRIFT

(11) **EP 3 272 819 B1**
(45) Veröffentlichungstag und Bekanntmachung des Hinweises auf die Patenterteilung: **05.12.2018**
(21) Anmeldenummer: 16180428.1
(22) Anmeldetag: 20.07.2016
(51) Int. Cl.: C09D 13/00

(54) **MINE, KREIDE ODER FARBTABLETTE FÜR SCHREIB-, MAL- UND/ODER KOSMETIKZWECKE, STIFT MIT EINER MINE UND VERFAHREN ZUR HERSTELLUNG EINER MINE, KREIDE ODER FARBTABLETTE**
REFILL, CHALK OR COLOUR TABLET FOR WRITING, PAINTING AND/OR COSMETIC PURPOSES, STICK WITH A REFILL AND METHOD FOR PRODUCING A REFILL, CHALK OR COLOUR TABLET
MINE, CRAIE OU PASTILLE COLORANTE DESTINEES A L'ECRITURE, AU DESSIN ET/OU AUX COSMETIQUES, CRAYON COMPRENANT UNE MINE ET PROCEDE DE PRODUCTION D'UNE MINE, D'UNE CRAIE OU D'UNE PASTILLE COLORANTE

(43) Veröffentlichungstag der Anmeldung: 24.01.2018
(73) Patentinhaber: Faber- Castell AG, 90546 Stein (DE)
(72) Erfinder: von Godin, Harald, 90522 Oberasbach (DE); Appel, Reiner, 90522 Oberasbach (DE); Lugert, Gerhard, 90431 Nürnberg (DE)
(74) Vertreter: Schlögl, Markus

(56) Entgegenhaltungen:
- EP-A2- 1 552 958
- DE-U1- 20 314 685
- KR-B1- 101 423 903
- US-A1- 2005 252 409

## Beschreibung

Die Erfindung betrifft eine Mine, Kreide oder Farbtablette für Schreib-, Mal- und/oder Kosmetikzwecke sowie einen Stift mit einer solchen Mine. Ferner betrifft die Erfindung ein Verfahren zur Herstellung einer Mine, Kreide oder Farbtablette.

Aus der EP 1 552 958 B1 sind Minen bekannt, die durch thermoplastische Verarbeitung einer als Bindemittel Zucker und/oder Zuckeralkohole sowie Cellulosederivate enthaltenden Minen-Ausgangsmasse hergestellt werden. Derartige Minen bzw. Minen-Ausgangsmassen sind nahezu wasserfrei und können daher über mehrere Wochen oder Monate gelagert werden. Ferner ist eine mikrobiologische Beeinträchtigung aufgrund des fehlenden Wassergehaltes kaum zu befürchten. Minen mit einem solchen Bindemittelsystem weisen zwar eine gute Bruchfestigkeit auf und zeichnen sich darüber hinaus durch einen sehr gleichmäßigen, homogenen Aufstrich auf einer Schreib- oder Malunterlage auf. Allerdings zeigen die Aufstriche auf Papier und Zeichenkarton der in der EP 1 552 958 beschriebenen thermoplastischen Minen lediglich eine befriedigende bis gute Aquarellierbarkeit. Der Farbauftrag lässt sich also nachträglich nicht ideal fein oder flächig vermalen und die Leuchtkraft kann sich daher nicht vollständig zu entfalten. Auch eine sehr gute Lichtechtheit der Farbaufstriche auf Papier oder Zeichenkarton konnte bisher nicht konstant gewährleistet werden. Bei Farbstiften, die von Künstlern eingesetzt werden, sind jedoch gerade hervorragende Aquarellierbarkeiten und sehr gute bis exzellente Lichtechtheiten gewünscht.

Es ist daher Aufgabe der Erfindung, eine Mine, Kreide oder Farbtablette für Schreib-, Mal- und/oder Kosmetikzwecke anzugeben, die insbesondere hinsichtlich der Aquarellierbarkeit und der Lichtechtheit verbessert ist. Ferner ist es Aufgabe der Erfindung ein Verfahren zur Herstellung einer Mine, Kreide oder Farbtablette sowie einen Stift mit einer Mine anzugeben.

Die erstgenannte Aufgabe wird gelöst mit einer Mine oder Kreide oder Farbtablette für Schreib-, Mal- und/oder Kosmetikzwecke mit den Merkmalen gemäß Anspruch 1. Danach sind in der Mine oder Kreide oder Farbtablette wenigstens ein Zucker und/oder Zuckeralkohol und/oder Pentaerythrit, wenigstens ein Cellulose- und/oder Stärkederivat und wenigstens ein Tensid enthalten, wobei das wenigstens eine Tensid ausgewählt ist aus der Gruppe der Schwefelsäure-Fettsäureester-Salze (=Fettalkoholsulfate) und/oder Fettsäuresulfoalkylester und/oder ethoxylierten Formen von Alkalimetallfettsäuresulfaten.

Im Folgenden wird vereinfachend vorwiegend auf eine Mine Bezug genommen, die Ausführungen gelten jedoch ebenso auch für eine Kreide oder eine Farbtablette. Die Mine oder Kreide oder Farbtablette ist insbesondere eine aquarellierbare Mine oder Kreide oder Farbtablette bzw. eine Aquarellfarbe.

Die Mine enthält somit als Bindemittel ein Bindemittelsystem, welches im Wesentlichen Zucker und/oder Zuckeralkohol und/oder Pentaerythrit und ein Cellulose- und/oder Stärkederivat enthält. Die Minengrundmasse bzw. die Minen-Ausgangsmasse, also der wenigstens eine Zucker und/oder Zuckeralkohol und/oder Pentaerythrit, das wenigstens eine Cellulose- und/oder Stärkederivat und das wenigstens ein Tensid sowie gegebenenfalls Pigmente und/oder Füllstoffe und/oder weitere Additive werden zur Herstellung der Mine bei erhöhter, ein Erweichen des Zuckers und/oder Zuckeralkohols und/oder Pentaerythrits bewirkenden Temperatur thermoplastisch verarbeitet. Dabei wird gegebenenfalls bei der Herstellung der Mine zur Förderung der Durchmischung der Ausgangsstoffe zugesetztes Wasser, wieder auf einen Anteil von maximal 2 Gew.-% reduziert. Die Mine selbst bzw. die Minengrundmasse ist wasserfrei und enthält höchstens 2 Gew.-% Wasser.

Die Mine ist somit aus einer die genannten Komponenten bzw. Ausgangsstoffe enthaltenen Minengrundmasse bzw. Minen-Ausgangsmasse hergestellt, die thermoplastisch verarbeitet und extrudiert wird, um die Ausgangsstoffe aufzuschmelzen und zu binden. Die daraus erhaltenen Minenstränge werden gegebenenfalls anschließend abgelängt. Nach dem Erkalten der Minenmasse liegt bereits die fertige Mine vor, ein aufwändiges Entfernen von Wasser oder Lösungsmitteln ist nicht erforderlich.

Bisher wurde der Einsatz bzw. die Zugabe von Tensiden bei herkömmlichen Minen bzw. Minenmassen, die Zucker und/oder Zuckeralkohole und / oder Pentaerythrit sowie wenigstens ein Cellulose- und/oder Stärkederivat sowie gegebenenfalls Pigmente und/oder Füllstoffe enthalten, vermieden, da Tenside, beispielsweise Silikon- oder Fluortenside, in Kombination mit einem solchen Bindemittelsystem bekanntermaßen zu einer Verschlechterung der mechanischen Stabilität der Mine oder Farbmine führen ohne anderweitige Vorteile mit sich zu bringen.

Überraschenderweise konnte jedoch festgestellt werden, dass bei Tensiden aus der Gruppe der Schwefelsäure-Fettsäureester-Salze und/oder Fettsäuresulfoalkyl-ester und/oder ethoxylierte Formen von Alkalimetallfettsäuresulfaten ein solcher Effekt nicht eintritt. Vielmehr konnten deutliche Verbesserungen der Aquarellierbarkeit der daraus hergestellten Minen erreicht werden, ohne dass eine spürbare Beeinträchtigung der mechanischen Stabilität eintritt. Außerdem traten durchwegs sehr gute bis exzellente Lichtechtheiten der Aufstriche auf. In den meisten Fällen wurden zudem sogar verbesserte Verarbeitungseigenschaften bei der Extrusion der Minengrundmasse und der daraus entwickelten Minen erreicht. Dies ist wohl darauf zurückzuführen, dass gerade die ausgewählten Tenside dazu geeignet sind, eine Verbindung oder Vermischung der Komponenten des Bindemittels, nämlich des Zuckers und/oder Zuckeralkohols und des Cellulose- und/oder Stärkederivates zu fördern.

Als Kationen der genannten Tenside dienen einwertige Metallkationen wie z.B. Kalium, Natrium, Lithium aber auch Ammonium. Vorzugsweise werden als Tenside bzw. als Schwefelsäure-Fettsäureester-Salze Natriumlaurylsulfat, Kaliumlaurylsulfat, Ammoniumlaurylsulfat, Salze wie Natriumcocoylisethionat oder Natriummyrethsulfat verwendet. Eine bevorzugt eingesetzte ethoxylierte Form eines Alkalimetallfettsäuresulfats stellt Natriumdodecylpoly(oxyethylen)sulfat dar.

Als Zucker wird vorzugsweise Saccharose und/oder Xylose eingesetzt. Um eine zuverlässige Verbindung des Zuckers mit dem Cellulose- und/oder Stärkederivat zu erreichen, ist jedoch während der thermoplastischen Verarbeitung bzw. dem Verschmelzen der beiden Komponenten oftmals ein erhöhter Druck im Extruder erforderlich, da zahlreiche Zucker wie beispielsweise Glucose, Fructose, Saccharose, Mannose sowie auch verschiedene Cellulose- und/oder Stärkederivate bei einer Verarbeitungstemperatur zwischen 100 und 220°C Zersetzungserscheinungen aufweisen. Dies kann durch die Verwendung von Zuckeralkoholen, insbesondere Mannit, Sorbit, Xylit, Adonit, Arabit, Dulcit oder Threit vermieden werden, die sich oberhalb von 100°C ohne Anwendung erhöhten Drucks zunächst wasserklar aufschmelzen lassen und Cellulosen oder Stärken bzw. deren Derivate überraschenderweise problemlos aufnehmen. Vor allem der Zuckeralkohol Mannit zeigt eine sehr gute Verarbeitbarkeit und hat sich daher als besonders vorteilhaft erwiesen. Die Verwendung eines einzigen Zuckers oder Zuckeralkohols ist ebenso denkbar, wie der Einsatz zweier oder mehrerer Zucker oder Zuckeralkohole in Kombination.

Als Cellulose- und/oder Stärkederivate haben sich Natriumcarboxymethylcellulose (NaCMC) und/oder Carboxymethylstärke (CMS) und/oder Natriumcarboxymethylstärke (NaCMS) als besonders geeignet erwiesen. Zur Vermischung bzw. Verbindung der beiden gemeinsam als Bindemittel wirkenden Komponenten, als der Zucker und/oder Zuckeralkohol und das Cellulose- und/oder Stärkederivat, ist eine Temperatur von etwa 80 bis 150°C ausreichend. Insbesondere durch Zugabe von Wasser während der Herstellung der Mine quellen die Cellulose- und/oder Stärkederivate auf und binden dadurch weitere Ausgangsstoffe.

Bei einer bevorzugten Ausführungsform enthält die Mine oder Kreide oder Farbtablette 1 bis 30 Gew.-%, insbesondere 1 bis 25 Gew.-% an Tensid. Wie bereits beschrieben, war es überraschend, dass die Verwendung eines Tensides aus der Gruppe der Schwefelsäure-Fettsäureester-Salze und/oder Fettsäuresulfoalkylester und/oder ethoxylierten Formen von Alkalimetallfettsäuresulfaten in dem vorliegenden Bindemittelsystem die Aquarellierbarkeit und Lichtechtheit der Minen deutlich verbessert, aber dennoch zu keiner nennenswerten Verschlechterung der mechanischen Stabilität der Minen führt. Diese Wechselwirkung bzw. dieser Effekt wird vorteilhafterweise dadurch gewährleistet, dass der Anteil des Tensides in der Mine bzw. in der Minengrundmasse 30 Gew.-%, insbesondere 25 Gew.-% nicht überschreitet.

Vorzugsweise enthält die Mine oder Kreide oder Farbtablette 4 bis 40 Gew.-%, insbesondere 4 bis 30 Gew.-% an Zucker und/oder Zuckeralkohol und/oder Pentaerythrit. Durch eine Begrenzung des Anteils an Zucker auf maximal 40 Gew.-%, insbesondere auf maximal 30 Gew.-% lässt sich eine Verschlechterung der Verarbeitbarkeit der Minengrundmasse und eine geringere Bruchfestigkeit der Mine zuverlässig vermeiden.

Ferner enthält die Mine bei einer bevorzugten Ausführungsform 0,5 bis 25 Gew.-%, insbesondere 0,5 bis 18 Gew.-% an Cellulosederivat und/oder Stärkederivat. Eine Begrenzung des Anteils an Cellulosederivat und/oder Stärkederivat auf maximal 25 Gew.-%, insbesondere auf maximal 18 Gew.-% stellt sicher, dass eine gute Verarbeitbarkeit, insbesondere eine gute Vermischung der Ausgangsstoffe bzw. insbesondere der beiden Bindemittel gewährleistet ist.

Durch Zugabe von einem oder mehreren Füllstoffen kann das Eigenschaftsprofil der Mine beeinflusst werden. Dabei hat sich die Zugabe eines Füllstoffes mit einem Anteil von 30 bis 80 Gew.-%, insbesondere mit einem Anteil von 40 bis 70 Gew.-% an der Minengrundmasse als vorteilhaft erwiesen. Als Füllstoff sind insbesondere Talkum, Kaolin, Bimsmehl, Calciumcarbonat, Bariumsulfat, Glimmer und/oder Titandioxid geeignet.

Sofern ein in der Mine enthaltenes Tensid bereits wachsartige Eigenschaften aufweist, kann gegebenenfalls auf den Zusatz von fett- oder wachsartigen Stoffen verzichtet werden. Beispielsweise zeigt Natriumcocoylisethionat bei der Verwendung als Tensid in Kombination mit den weiteren Bestandteilen der Mine bzw. der Minengrundmasse solche wachsartigen Eigenschaften. Um das Eigenschaftsprofil der Mine, etwa deren Abstrichverhalten jedoch weiter variieren zu können, enthält die Minengrundmasse bei einer bevorzugten Variante wenigstens einen fett- oder wachsartigen Stoff, insbesondere mit einem Anteil von 0,1 bis 30 Gew.-%. Der fett- oder wachsartige Stoff ist dabei vorzugsweise wenigstens eine Fettsäure und/oder ein Fettsäurederivat und/oder ein Wachs. Durch die Verwendung fett- oder wachsartiger Stoffe, die sowohl lipophile als auch hydrophile Eigenschaften besitzen, lassen sich wasserlösliche bzw. wasserquellbare Minen herstellen. Dadurch werden somit Minen erhalten, die in ihren Eigenschaften den bekannten sogenannten "Post-Wax-Minen" entsprechen, welche nach ihrer Herstellung in geschmolzenes Wachs oder Öl eingetaucht werden. Dieser zusätzliche Arbeitsschritt entfällt hier, was die Herstellung der Minen vereinfacht, insbesondere die zur Herstellung der Mine erforderliche Zeit verkürzt. Als solche fett- oder wachsartigen Stoffe kommen beispielsweise Fettsäuren wie Stearinsäure, Paraffinwachse, Japanersatzwachs oder Etoxy (80) Cetostearyl Alcohol, andere Fettsäurederivate oder Hydrocarbonwachs zum Einsatz.

Um farbige Minen oder Kreiden oder Farbtabletten zu erhalten, enthält die Minengrundmasse wenigstens ein Farbmittel, insbesondere ein anorganisches und/oder organisches Farbpigment, insbesondere mit einem Anteil von 0,5 bis 20 Gew.-% enthalten ist.

Bei einer weiteren bevorzugten Variante, enthält die Mine wenigstens ein anorganisches und/oder organisches Farbpigment und wenigstens einen wasserlöslichen Farbstoff in Kombination. Der Zusatz von wasserlöslichen Farbstoffen reduziert zwar die ausgezeichnete Lichtechtheit, aber dadurch wird die Herstellung von sogenannten Kopierstiftminen bzw. der Einsatz der Mine in einem Kopierstift ermöglicht.

Je nach Bedarf kann die Mine weitere Additive, wie beispielsweise Gleitmittel oder Emulgatoren enthalten, die der Minengrundmasse bei der Herstellung der Mine zugefügt werden, um das Eigenschaftsprofil der Mine weiter zu variieren.

Die zweitgenannte Aufgabe wird gelöst mit einem Stift mit den Merkmalen gemäß Anspruch 15, welcher eine Mine mit den vorstehend beschriebenen Merkmalen umfasst. Der Stift weist dabei insbesondere eine Ummantelung aus Holz oder aus einem Holzersatzwerkstoff auf, welcher die Mine umhüllt. Als Holzersatzwerkstoff wird anstelle von natürlichem Holz beispielsweise ein sogenanntes Wood-Plastic-Compound eingesetzt, sodass der Stift beispielsweise in einfacher Weise durch Co-Extrusion der Mine und der Ummantelung hergestellt werden kann.

Die drittgenannte Aufgabe wird gelöst durch ein Verfahren zur Herstellung einer Mine oder einer Kreide oder einer Farbtablette mit den Merkmalen gemäß Anspruch 16. Gemäß dem Verfahren werden die zur Herstellung der Mine erforderlichen Ausgangsstoffe bzw. die in der Minengrundmasse enthaltenen Stoffe, also der wenigstens eine Zucker und/oder Zuckeralkohol und/oder Pentaerythrit, das wenigstens ein Cellulose- und/oder Stärkederivat und das wenigstens eine Tensid vermischt und bei erhöhter, ein Erweichen des Zuckers und/oder des Zuckeralkohols und/oder des Pentaerythrits bewirkender Temperatur thermoplastisch verarbeitet bzw. vermischt und miteinander verbunden. Gegebenenfalls werden weitere Stoffe, wie beispielsweise Füllstoffe oder ein fett- oder wachsartiger Stoff beigefügt und gemeinsam mit den vorstehend genannten Ausgangsstoffen thermoplastisch verarbeitet. Die daraus erhaltene Minengrundmasse wird zu Minen oder Kreidesträngen extrudiert und/oder die daraus erhaltene Minengrundmasse wird zu Farbtabletten ausgeformt. Die wenigstens ein Tensid, welches ausgewählt ist aus der Gruppe der Schwefelsäure-Fettsäureester-Salze und/oder Fettsäuresulfoalkylester und/oder ethoxylierten Formen von Alkalimetallfettsäuresulfaten enthaltende Minengrundmasse lässt sich im Vergleich zu herkömmlichen Minen, welche kein Tensid aufweisen, besser thermoplastisch verarbeiten. Das Tensid führt zu einer besseren Vermischbarkeit und innigeren Verbindung der Ausgangsstoffe.

Vorzugsweise wird den Ausgangsstoffen Wasser zugegeben, welches gemeinsam mit dem wenigstens einen Zucker und/oder Zuckeralkohol und/oder Pentaerythrit, dem wenigstens einen Cellulose- und/oder Stärkederivat und dem wenigstens einen Tensid thermoplastisch verarbeitet wird, wobei der Anteil des Wassers während der thermoplastischen Verarbeitung, etwa bei der Compoundherstellung im Extruder, auf höchstens 2 Gew.-% reduziert wird. Mit anderen Worten: Das vorhandene Wasser, welches den Ausgangsstoffen beispielsweise mit einem Anteil von 5 bis 30 Gew.-% zugegeben wird, um den Vermischungs- bzw. Compoundierungsprozess des Zuckers und/oder Zuckeralkohols und/oder Pentaerythrits, des wenigstens einen Cellulose- und/oder Stärkederivats und des wenigstens einen Tensids sowie gegebenenfalls weiteren Stoffen zu fördern, wird bis auf einen Restgehalt von kleiner gleich 2 Gew.-% verdunstet, sodass dann ein Aufschmelzen des Zuckers oder Zuckeralkohols oder Pentaerythrits, welches in wässriger Lösung naturgemäß nicht erreichbar ist, möglich ist. Die extrudierte oder ausgeformte Minengrundmasse weist durch die Reduzierung des Wassergehaltes einen geringen Feuchtigkeitsgehalt auf, sodass nach dem Erkalten der Minengrundmasse bereits die fertige Mine vorliegt. Ein aufwändiges Entfernen von Wasser oder sonstigen Lösungsmitteln entfällt.

Nachfolgend sind vier Beispielrezepturen für eine Mine aufgeführt. Die Ausgangssubstanzen bzw. Rohmaterialien werden zunächst in einem Schnellmischer oder in einem Extruder bei einer Temperatur von 80 bis 150°C vermischt. Zur Beschleunigung des Vermischungs- bzw. Homogenisierungsprozesses wird ein Wasseranteil von 15 bis 20 Gew.-% zugegeben und während des Mischvorgangs im Schnellmischer oder Extruder bis auf eine Restfeuchtigkeit von maximal 2 Gew.-% wieder verdunstet. Die erhaltene Minengrundmasse wird in einem Schneckenextruder bei etwa 140 bis 180°C extrudiert und anschließend zu Minen abgelängt. Die so hergestellten Minen oder Kreiden weisen einen Durchmesser zwischen 2,6 und 6,0 mm auf.

### Beispiel 1:

Aquarellierbare grüne Farbmine oder Kreide:

| | |
|---|---|
| Mannit | 6,0 Gew. % |
| NaCMC ¹⁾ | 4,0 Gew. % |
| Natriumcocoylisethionat (CAS 61789-32-0) | 20,0 Gew. % |
| Kaolin | 56,4 Gew. % |
| Titandioxid | 5,0 Gew. % |
| Pigment Yellow 3 | 1,8 Gew. % |
| Pigment Yellow 1 | 0,9 Gew. % |
| Pigment Blue 15 | 1,35 Gew. % |
| Pigment Green 7 | 4,55 Gew. % |

Eine Mine oder Kreide bzw. ein Stift mit einer Mine mit einer Zusammensetzung entsprechend Beispiel 1 weist eine sehr gute Verarbeitbarkeit bzw. Extrudierbarkeit auf. Dies ist insbesondere auf die Verwendung von Mannit als Zuckeralkohol sowie den hohen Anteil von 20 Gew.-% an dem eingesetzten Tensid (Natriumcocoylisethionat) zurückzuführen. Ferner zeigt die Mine eine exzellente Aquarellierbarkeit und Lichtechtheit.

### Beispiel 2:

Aquarellierbare blaue Farbmine oder Kreide:

| | |
|---|---|
| Sorbit | 6,7 Gew. % |
| Carboxymethylstärke ²⁾ | 4,5 Gew. % |
| Natriummyrethsulfate (CAS 25446-80-4) | 10,0 Gew. % |
| Hydrocarbonwachs | 6,8 Gew. % |
| Bimsmehl | 63,2 Gew. % |
| Titandioxid | 1,0 Gew. % |
| Pigment Blue 15 | 7,2 Gew. % |
| Pigment Red 146 | 0,6 Gew. % |

Bei einer Mine oder Kreide bzw. einem Stift mit einer Mine mit einer Zusammensetzung entsprechend Beispiel 2 wurde der Anteil an Tensid, vorliegend Natriummyrethsulfat im Vergleich zu Beispiel 1 reduziert. Dennoch zeigt sich bei der Herstellung der Mine weiterhin eine ausreichend gute Verarbeitbarkeit und Extrudierbarkeit bei vergleichbarer Aquarellierbarkeit und Lichtechtheit.

### Beispiel 3:

Aquarellierbare rote Farbmine oder Kreide:

| | |
|---|---|
| Xylit | 7,0 Gew. % |
| Carboxymethylstärke ²⁾ | 5,0 Gew. % |
| Natriumlaurylsulfoacetat (CAS 1847-58-1) | 8,0 Gew. % |
| Sorbitanmonostearat | 8,0 Gew. % |
| Talkum | 62,6 Gew. % |
| Titandioxid | 1,0 Gew. % |
| Pigment Red 254 | 7,0 Gew. % |
| Pigment Yellow 3 | 1,4 Gew. % |

Bei einer Mine oder Kreide bzw. einem Stift mit einer Mine mit einer Zusammensetzung entsprechend Beispiel 3 wurde als Tensid Natriumlaurylsulfoacetat eingesetzt und dessen Anteil im Vergleich zu Beispiel 1 und 2 weiter reduziert, nämlich auf 8 Gew.-%. Auch hier sind jedoch Aquarellierbarkeit und Lichtechtheit sowie Verarbeitbarkeit noch zufriedenstellend.

### Beispiel 4:

Aquarellierbare orange Farbmine oder Kreide:

| | |
|---|---|
| Mannit | 17,4 Gew. % |
| Carboxymethylstärke ²⁾ | 2,3 Gew. % |
| Natriumlaurylsulfat (CAS 151-21-3) | 15,0 Gew. % |
| Hydrocarbonwachs | 4,3 Gew. % |
| Kaolin | 50,6 Gew. % |
| Titandioxid | 6,0 Gew. % |
| Pigment Yellow 74 | 3,6 Gew. % |
| Pigment Orange 34 | 0,8 Gew. % |

Bei einer Mine oder Kreide bzw. einem Stift mit einer Mine mit einer Zusammensetzung entsprechend Beispiel 4 wurde als Tensid Natriumlaurylsulfat eingesetzt. Eine derartige Mine oder Kreide zeigt insbesondere eine gute Aquarellierbarkeit.

### Produktbezeichnungen/Hersteller:

1) Dow Wolff Cellulosics GmbH, D-29699 Bomlitz
2) Südstärke GmbH, D-86529 Schrobenhausen

## Patentansprüche

1. Mine oder Kreide oder Farbtablette für Schreib-, Mal- und/oder Kosmetikzwecke **dadurch gekennzeichnet, dass** wenigstens ein Zucker und/oder Zuckeralkohol und/oder Pentaerythrit, wenigstens ein Cellulose- und/oder Stärkederivat und wenigstens ein Tensid enthalten sind, wobei das wenigstens eine Tensid ausgewählt ist aus der Gruppe der Schwefelsäure-Fettsäureester-Salze und/oder Fettsäuresulfoalkylester und/oder ethoxylierten Formen von Alkalimetallfettsäuresulfaten.

2. Mine oder Kreide oder Farbtablette nach Anspruch 1, **dadurch gekennzeichnet, dass** als Tensid Natriumlaurylsulfat, Kaliumlaurylsulfat, Ammoniumlaurylsulfat, Natriumcocoylisethionat, Natriummyrethsulfat und/oder Natriumdodecylpoly(oxyethylen)sulfat enthalten ist.

3. Mine oder Kreide oder Farbtablette nach einem der Ansprüche 1 oder 2, **dadurch gekennzeichnet, dass** als Zucker Saccharose und/oder Xylose enthalten sind.

4. Mine oder Kreide oder Farbtablette nach einem der vorhergehenden Ansprüche, **dadurch gekennzeichnet, dass** als Zuckeralkohol Mannit, Sorbit, Xylit, Adonit, Arabit, Dulcit oder Threit enthalten sind.

5. Mine oder Kreide oder Farbtablette nach einem der vorhergehenden Ansprüche, **dadurch gekennzeichnet, dass** als Cellulose- und/oder Stärkederivat Natriumcarboxymethylcellulose und/oder Carbocymethylstärke und/oder Natriumcarboxymethylstärke enthalten sind.

6. Mine oder Kreide oder Farbtablette nach einem der vorhergehenden Ansprüche, **dadurch gekennzeichnet, dass** 1 bis 30 Gew.-%, insbesondere 1 bis 25 Gew.-% an Tensid enthalten sind.

7. Mine oder Kreide oder Farbtablette nach einem der vorhergehenden Ansprüche, **dadurch gekennzeichnet, dass** 4 bis 40 Gew.-%, insbesondere 4 bis 30 Gew.-% an Zucker und/oder Zuckeralkohol und/oder Pentaerythrit enthalten sind.

8. Mine oder Kreide oder Farbtablette nach einem der vorhergehenden Ansprüche, **dadurch gekennzeichnet, dass** 0,5 bis 25 Gew.-%, insbesondere 0,5 bis 18 Gew.-% an Cellulosederivat und/oder Stärkederivate enthalten sind.

9. Mine oder Kreide oder Farbtablette nach einem der vorhergehenden Ansprüche, **dadurch gekennzeichnet, dass** sie wenigstens einen Füllstoff mit einem Anteil von 30 bis 80 Gew.-%, insbesondere mit einem Anteil von 40 bis 70 Gew.-% enthält.

10. Mine oder Kreide oder Farbtablette nach Anspruch 9, **dadurch gekennzeichnet, dass** als Füllstoff Talkum, Kaolin, Bimsmehl, Calciumcarbonat, Bariumsulfat, Glimmer und/oder Titandioxid enthalten ist.

11. Mine oder Kreide oder Farbtablette nach einem der vorhergehenden Ansprüche, **dadurch gekennzeichnet, dass** sie wenigstens einen fett- oder wachsartigen Stoff, insbesondere mit einem Anteil von 0,1 bis 30 Gew.-% enthält.

12. Mine oder Kreide oder Farbtablette nach Anspruch 11, **dadurch gekennzeichnet, dass** der wenigstens eine fett-oder wachsartige Stoff eine Fettsäure und/oder ein Fettsäurederivat und/oder ein Wachs enthält.

13. Mine oder Kreide oder Farbtablette nach einem der vorhergehenden Ansprüche, **dadurch gekennzeichnet, dass** sie wenigstens ein Farbmittel, insbesondere ein anorganisches und/oder organisches Farbpigment, insbesondere mit einem Anteil von 0,5 bis 20 Gew.-% enthalten ist.

14. Mine oder Kreide oder Farbtablette nach Anspruch 13, **dadurch gekennzeichnet, dass** sie wenigstens ein anorganisches und/oder organisches Farbpigment und wenigstens einen wasserlöslichen Farbstoff enthält.

15. Stift mit einer Mine nach einem der Ansprüche 1 bis 14, insbesondere mit einer Ummantelung aus Holz oder aus einem Holzersatzwerkstoff.

16. Verfahren zur Herstellung einer Mine oder einer Kreide oder einer Farbtablette nach einem der Ansprüche 1 bis 14, **dadurch gekennzeichnet, dass** der wenigstens eine Zucker und/oder Zuckeralkohol und/oder Pentaerythrit, das wenigstens ein Cellulose- und/oder Stärkederivat und das wenigstens eine Tensid bei erhöhter, ein Erweichen des Zuckers und/oder des Zuckeralkohols und/oder des Pentaerythrits bewirkender Temperatur thermoplastisch verarbeitet werden, und die daraus erhaltene Minengrundmasse zu Minen oder Kreidesträngen extrudiert und/oder die daraus erhaltene Minengrundmasse zu Farbtabletten ausgeformt wird.

17. Verfahren nach Anspruch 16, **dadurch gekennzeichnet, dass** Wasser zugegeben wird, welches gemeinsam mit dem wenigstens einen Zucker und/oder Zuckeralkohol und/oder Pentaerythrit, dem wenigstens einen Cellulose- und/oder Stärkederivat und dem wenigstens einen Tensid vermischt wird und wobei der Anteil des Wassers während der thermoplastischen Verarbeitung auf höchstens 2 Gew.-% reduziert wird.

## Claims

1. Lead or chalk or colour palette for writing, drawing or cosmetic purposes, **characterised in that** at least one sugar and/or sugar alcohol and/or pentaerythritol, at least one cellulose derivate and/or starch derivative and at least one surfactant are contained, wherein the at least one surfactant is selected from the group of sulphuric acid fatty acid ester salts and/or fatty acid sulphoalkyl esters and/or ethoxylated forms of alkali metal fatty acid sulphates.

2. Lead or chalk or colour palette according to claim 1, **characterised in that** sodium lauryl sulphate, potassium lauryl sulphate, ammonium lauryl sulphate, sodium cocoyl isethionate, sodium myreth sulphate and/or sodium dodecylpoly(oxyethylene) sulphate is contained as the surfactant.

3. Lead or chalk or colour palette according to one of claims 1 or 2, **characterised in that** sucrose and/or xylose are contained as sugar.

4. Lead or chalk or colour palette according to one of the preceding claims, **characterised in that** mannitol, sorbitol, xylitol, adonitol, arabitol, dulcitol or threitol are contained as sugar alcohol.

5. Lead or chalk or colour palette according to one of the preceding claims, **characterised in that** sodium carboxymethyl cellulose and/or carboxymethyl starch and/or sodium carboxymethyl starch are contained as cellulose and/or starch derivate.

6. Lead or chalk or colour palette according to one of the preceding claims, **characterised in that** 1 to 30% by weight, in particular 1 to 25% by weight of surfactant are contained.

7. Lead or chalk or colour palette according to one of the preceding claims, **characterised in that** 4 to 40% by weight, in particular 4 to 30% by weight of sugar and/or sugar alcohol and/or pentaerythritol are contained.

8. Lead or chalk or colour palette according to one of the preceding claims, **characterised in that** 0.5 to 25% by weight, in particular 0.5 to 18% by weight of cellulose derivate and/or starch derivate are contained.

9. Lead or chalk or colour palette according to one of the preceding claims, **characterised in** it contains at least one filler having a proportion of 30 to 80% by weight, in particular having a proportion of 40 to 70% by weight.

10. Lead or chalk or colour palette according to claim 9, **characterised in that** talcum, kaolin, pumice powder, calcium carbonate, barium sulphate, mica and/or titanium dioxide is contained as the filler.

11. Lead or chalk or colour palette according to one of the preceding claims, **characterised in that** it contains at least one fatty or waxy substance, in particular having a proportion of 0.1 to 30% by weight.

12. Lead or chalk or colour palette according to claim 11, **characterised in that** the fatty or waxy substance contains a fatty acid and/or a fatty acid derivate and/or a wax.

13. Lead or chalk or colour palette according to one of the preceding claims, **characterised in that** it is contained at least one colourant, in particular an inorganic and/or organic colour pigment, in particular having a proportion of 0.5 to 20 % by weight.

14. Lead or chalk or colour palette according to claim 13, **characterised in that** it contains at least one inorganic and/or organic colour pigment and at least one water-soluble colourant.

15. Pencil having a lead according to one of claims 1 to 14, in particular having a casing made of wood or made of a wood substitute material.

16. Method for producing a lead or a chalk or a colour palette according to one of claims 1 to 14, **characterised in that** the at least one sugar and/or sugar alcohol and/or pentaerythritol, the at least one cellulose derivate and/or starch derivate and the at least one surfactant are thermoplastically processed at an increased temperature, said temperature causing softening of the sugar and/or the sugar alcohol and/or the pentaerythritol, and the lead base material obtained therefrom is extruded into leads or chalk strands and/or the lead base material obtained therefrom is shaped into colour palettes.

17. Method according to claim 16, **characterised in that** water is added which is mixed together with the at least one sugar and/or sugar alcohol and/or pentaerythritol, the at least one cellulose derivate and/or starch derivate and the at least one surfactant, and wherein the proportion of the water during the thermoplastic processing is reduced to 2% by weight at most.

## Revendications

1. Mine ou craie ou pastille colorante destinée à l'écriture, au dessin et/ou à des fins cosmétiques, **caractérisée en ce que** sont contenus au moins un sucre et/ou un alcool-sucre et/ou du pentaérythrite, au moins un dérivé de cellulose et/ou d'amidon et au moins un agent tensioactif, dans lequel le au moins un agent tensioactif est choisi dans le groupe des sels d'ester d'acide gras d'acide sulfurique et/ou des esters sulfoalcoyliques d'acide gras et/ou des formes éthoxylées de sulfates d'acide gras de métal alcalin.

2. Mine ou craie ou pastille colorante suivant la revendication 1, **caractérisée en ce qu'**est contenu, comme agent tensioactif, du laurylsulfate de sodium, du laurylsulfate de potassium, du laurylesulfate d'ammonium, du cocoyliséthionate de sodium, du myréthsulfate de sodium et/ou du dodécylpoly (oxyéthylène)sulfate de sodium.

3. Mine ou craie ou pastille colorante suivant la revendication 1 ou 2, **caractérisée en ce qu'**est contenu, comme sucre, du saccharose et/ou du xylose.

4. Mine ou craie ou pastille colorante suivant l'une des revendications précédentes, **caractérisée en ce qu'**est contenu, comme alcool-sucre, du mannite, du sorbite, du xylite, de l'adonite, de l'arabite, du dulcite ou du thréite.

5. Mine ou craie ou pastille colorante suivant l'une des revendications précédentes, **caractérisée en ce qu'**est contenu, comme dérivé de cellulose et/ou d'amidon, de la carboxyméthylcellulose de sodium et/ou du carboxyméthylamidon et/ou du carboxyméthylamidon de sodium.

6. Mine ou craie ou pastille colorante suivant l'une des revendications précédentes, **caractérisée en ce qu'**est contenu de 1 à 30% en poids, notamment de 1 à 25% en poids, d'agent tensioactif.

7. Mine ou craie ou pastille colorante suivant l'une des revendications précédentes, **caractérisée en ce qu'**est contenu de 4 à 40% en poids, notamment de 4 à 30% en poids, de sucre et/ou d'alcool-sucre et/ou de pentaérythrite.

8. Mine ou craie ou pastille colorante suivant l'une des revendications précédentes, **caractérisée en ce qu'**est contenu de 0,5 à 25% en poids, notamment de 0,5 à 18% en poids, de dérivé de cellulose et/ou de dérivé d'amidon.

9. Mine ou craie ou pastille colorante suivant l'une des revendications précédentes, **caractérisée en ce qu'**elle contient au moins une charge en une proportion de 30 à 80% en poids, notamment en une proportion de 40 à 70% en poids.

10. Mine ou craie ou pastille colorante suivant la revendication 9, **caractérisée en ce qu'**est contenu, comme charge, du talc, du kaolin, de la poudre de pierre ponce, du carbonate de calcium, du sulfate de baryum, du mica et/ou du dioxyde de titane.

11. Mine ou craie ou pastille colorante suivant l'une des revendications précédentes, **caractérisée en ce qu'**elle contient au moins une matière grasse ou de type cire, notamment en une proportion de 0,1 à 30% en poids.

12. Mine ou craie ou pastille colorante suivant la revendication 11, **caractérisée en ce que** la au moins une matière grasse ou de type ou cire contient un acide gras et/ou un dérivé d'acide gras et/ou une cire.

13. Mine ou craie ou pastille colorante suivant l'une des revendications précédentes, **caractérisée en ce qu'**elle contient au moins un colorant, notamment un pigment coloré minéral et/ou organique, notamment en une proportion de 0,5 à 20% en poids.

14. Mine ou craie ou pastille colorante suivant la revendication 13, **caractérisée en ce qu'**elle contient au moins un pigment coloré minéral et/ou organique et au moins un colorant soluble dans l'eau.

15. Crayon ayant une mine suivant l'une des revendications 1 à 14, ayant notamment un enrobage en bois ou en un matériau de substitution du bois.

16. Procédé de fabrication d'une mine ou d'une craie ou d'une pastille colorant suivant l'une des revendications 1 à 14, **caractérisé en ce que** l'on traite thermoplastiquement le au moins un sucre et/ou l'alcool-sucre et/ou le pentaérythrite, le au moins un dérivé de cellulose et/ou d'amidon et le au moins un agent tensioactif a une température élevée provoquant un amolissement du sucre et/ou de l'alcool-sucre et/ou du pentaérythrite et on extrude la composition de base de mine ainsi obtenue en des mines ou en des cordons de craie et/ou on forme la composition de base de mine ainsi obtenue en des pastilles colorantes.

17. Procédé suivant la revendication 16, **caractérisé en ce que** l'on ajoute de l'eau que l'on mélange conjointement au au moins un sucre et/ou un alcool-sucre et/ou au pentaérythrite, le au moins un dérivé de cellulose et/ou d'amidon et le au moins un agent tensioactif, et dans lequel on réduit la proportion de l'eau pendant le traitement thermoplastique à au plus 2% en poids.
